# EUROPEAN PATENT SPECIFICATION

(11) **EP 1 033 935 B1**
(45) Date of publication and mention of the grant of the patent: **29.03.2006**
(21) Application number: 98944079.7
(22) Date of filing: 22.09.1998
(51) Int. Cl.: A61B 5/113

(54) **RESPIRATORY MOVEMENT SENSOR DEVICE**
ATMUNGSBEWEGUNGSFÜHLER
APPAREIL DE DETECTION DU MOUVEMENT RESPIRATOIRE

(30) Priority: 24.09.1997 GB 9720175
(43) Date of publication of application: 13.09.2000
(73) Proprietor: KinderTec Limited, Cowley Road Cambridge CB4 4WS (GB)
(72) Inventor: LIU, Chi, Cheung Eagletron Telecommunications Ltd., Hong Kong (CN); GOOCH, David, Hardwick,Cambridge CB3 7QS (GB)
(74) Representative: Lawrence, John
(86) International application number: PCT/GB1998/002861
(87) International publication number: WO 1999/015076

(56) References cited:
- WO-A-91/19456
- FR-A- 2 742 648
- GB-A- 2 261 290

## Description

### Field of the invention

This invention relates to a sensor device for detecting movement of a person, and in particular a sensor device on which a person lies, where this movement can be breathing, heart beat, twitches or the like. The invention is also concerned with a system for monitoring the respiratory movement of a person.

### Background of the invention

The use of sensor devices to detect respiratory movement in children is well known. Such devices can be in the form of belts or mats, and typically the device includes a piezo-electric sensor which produces an electrical signal in response the movement of a breathing child. This electrical signal is monitored to determine if the child stops breathing. Known sensor mats have a limited sensitivity, in particular if the child rolls to an outer edge of the mat, and thus they may not always provide an accurate indication of whether breathing is occurring.

We are aware of the PCT application published as WO91/19456, which discloses a movement sensor device in which two panels are mounted for movement relative to one another. Any relative movement of the panels is detected due to the action of a protuberance on the top panel pressing against a lever attached to a piezo-electric sensor on a lower panel. This document forms the pre-characterising portion of claims 1 and 2.

We are also aware of GB 2 261 290, which discloses a fabric patch with piezo-electric devices there within, which senses the movement of the wearer (typically an infant); and FR 2 274 648, which discloses a movement sensor for an infant's bed comprising two plates and a piezo-electric sensor therebetween.

### Disclosure of the invention

According to a first aspect of the invention, there is provided a sensor device for detecting respiratory movement in a person, the device comprising a lower panel, an upper panel resiliently mounted on the lower panel and guided for limited relative movement in a direction towards or away from the lower panel, and sensing means positioned between the panels and operative to produce an electrical signal representative of movement of the upper panel with respect to the lower panel, and ribs are provided to streng then the upper panel and improve rigidity. In such a manner the upper panel floats on the lower panel whilst ensuring that the respective areas of the upper panel and the lower panel are maintained in registration with each other. Thus in use, with a person resting on the upper panel, the upper panel is free to resiliently deflect in response to the person's movement.

In another aspect of the invention, the sensor device comprises the features as defined in claim 2.

The sensor device may be encased in plastics material which provides a substantially waterproof seal about the device so as to prevent ingress of water and urine. Such a casing improves the long term reliability of the device as degradation of the sensing means due to corrosion is substantially reduced.

Preferably, each of the lower panel and upper panel is moulded from a rigid or semi-rigid plastics material such as ABS, and this moulding may be performed by injection moulding. Where the upper panel and the lower panel are moulded from plastics material, provision of ribs on the internal surface of the upper panel is of particular advantage as this strengthens the upper panel and improves rigidity. The ribs also prevent long term deformation of the upper panel as without the provision of ribs, the upper panel may irreversibly deform in use until it ceases to move in response to movement of a person.

Where the panels are manufactured by injection moulding, the ribs are integrally moulded into the upper panel. The ribs are then of further advantage in that they ensure the panel remains flat after injection moulding. Desirably the ribs are moulded to form channels where electrical wiring between the individual sensors may be placed.

In a preferred embodiment of the first aspect of the present invention, one of the panels has a projecting peripheral lip which locates within a peripheral rim on the other panel to provide the guidance. The peripheral lip and the peripheral rim may include corresponding portions which interengage to provide a snap-fit connection between the upper and lower panel.

A plurality of resilient pads, disposed between the panels and spaced over the overlapping area of the panels, preferably provides the resilient mounting of the upper panel on the lower panel, and the sensing means may be a plurality of piezo-electric sensors engaged by the pads. Typically each piezo-electric sensor overlies one resilient pad when the panels are interengaged. In use, as the upper panel moves towards or away from the lower panel in response to the movement of a person, the resulting variable weight distribution across the panel is transmitted to the resilient pads which compress or expand. This causes the piezo-electric sensor to generate an electrical signal in response to the movement of the person.

The use of a plurality of sensors increases the sensitivity of the sensor device, and advantageously five sensors are provided, wherein one sensor is attached proximal to the centre of the inner surface of the upper panel, and four sensors are each positioned at spaced apart locations near the edge of the inner surface of the upper panel. Thus the sensors may be electrically wired together on one panel. Where the upper panel is rectangular or square in configuration, the preferred positioning of the plurality of sensors results in an "X" configuration of the sensors. This positioning of sensors at discrete spaced apart locations over the inner surface of the upper panel provides improved sensitivity, electrically connecting the sensors in parallel with each other may also occur. As the highest bending amplitude occurs in the middle of the upper panel, only one sensor needs to be placed in the middle area, and the positioning of the other four sensors at the corner positions of the upper panel compensates for low sensitivities of the mat near the edges and corners. This is of advantage when the device is in use as if a person moves substantially off the sensor device, at least one of the edge sensors is still sufficiently close to the person to detect movement.

In use, the sensor device is placed below a person, typically a small child, and respiratory movement of the person results in movement of the upper panel, thereby causing the sensor means to generate an electrical signal. The electrical signal is then typically communicated to a detection means which monitors the movement of the person and thus the sensor device may be used, for example, to monitor the respiratory movement of a child. Thus in accordance with a further aspect of the invention, there is provided a system for translating the movement of a person into a signal for monitoring the respiratory movement, comprising a sensor device as aforesaid, a transmitter, and a receiver, wherein the electrical signals produced from the sensor device are communicated to the transmitter, the transmitter then communicating these signals to a receiver.

The receiver is typically remote from the sensor device and transmitter. The transmitter may also be remote from the sensor device, or alternatively may be provided with a direct connection to the sensor device. Thus the transmitter may be directly wired to the sensor device or may communicate with the sensor device by telemetry. Preferably the receiver is provided with an LCD display for visual display of information derived from the electrical signals resulting from the sensor device and from other sources.

A sensor device forming a preferred embodiment of the invention will now be described, by way of example, with reference to the accompanying drawings, in which:
Figure 1 is a diagrammatic perspective view of the exemplary sensor device when in use to measure respiratory movement of a child;
Figure 2 is a plan view of the internal surface of an upper panel forming part of the sensor device;
Figure 3 is a sectional view on the line III-III of Figure 2;
Figure 4 is a plan view of the internal surface of a lower panel forming part of the sensor device;
Figure 5 is a sectional view on the line V-V of Figure 4;
Figure 6 is a side view of the upper panel and the lower panel when assembled to form the sensor device;
Figure 7 is an exploded view of the parts in Figure 6 showing a side view of the upper and lower panel prior to assembly;
Figure 8 is a fragmentary view to explain how individual sensors within the sensor device operate;
Figure 9 is a diagrammatic sectional view along the line IX-IX of Figure 2 showing one exemplary method of mounting a sensor;
Figure 10 is a diagrammatic sectional view along the line IX-IX of Figure 2 showing a modification of the upper panel for mounting a sensor;
Figure 11 is a plan view along the line IX-IX of Figure 2, without the sensor in position, showing a modification of the upper panel for mounting a sensor; and
Figure 12 is a schematic view of different numbers of sensors devices connected to increase sensing area.

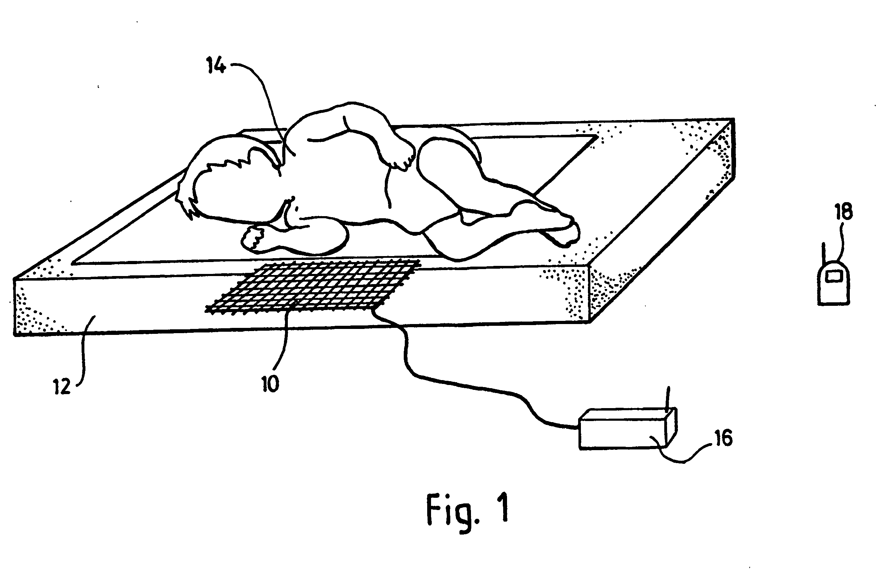

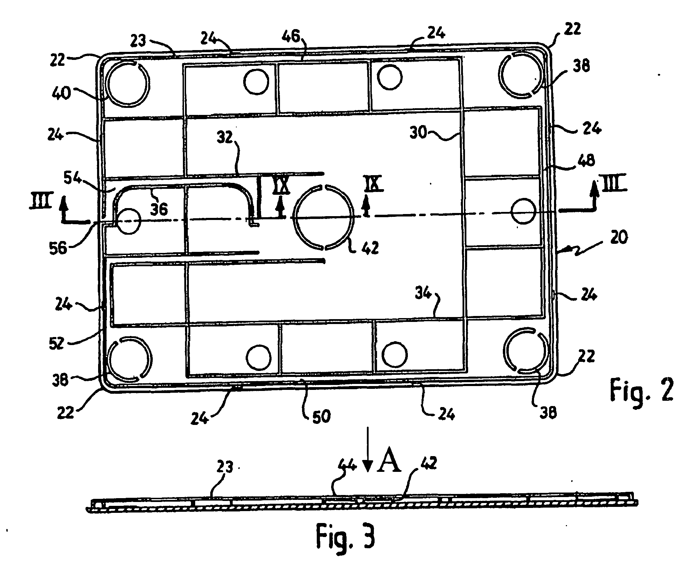

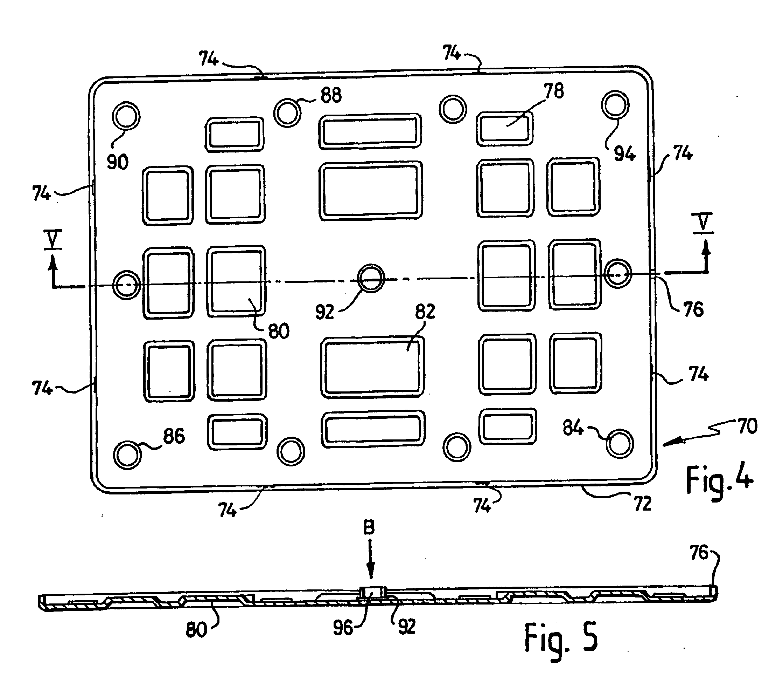

In Figure 1, the sensor device 10 comprises an upper panel 20, a lower panel 70 (as shown in Figures 2 and 4 respectively) and sensing means, and is shown positioned under a cot mattress 12 on which a child 14 is resting. The sensor device 10 covers a smaller area than the mattress and can be encased in plastics material, such as PVC, which provides a substantially waterproof seal about the device 10 so as to prevent ingress of water and urine into the device 10. As the child breathes, the related body movement is detected by the sensing means which produces an electrical signal related to the movement of the child. This signal is passed to a signal processing unit 16 either by wire connection or very short range radio frequency telemetry and then by telemetry to a portable receiver 18 which detects and displays the breathing response over time. The signal processing unit 16 may also store data for addressing at a later stage, for example, where a hospital is monitoring a child in a home environment.

The system illustrated in Figure 1 is suitable for home use and, by use of very short range telemetry, can also be used in areas with a number of sensitive electrical devices, such as hospitals.

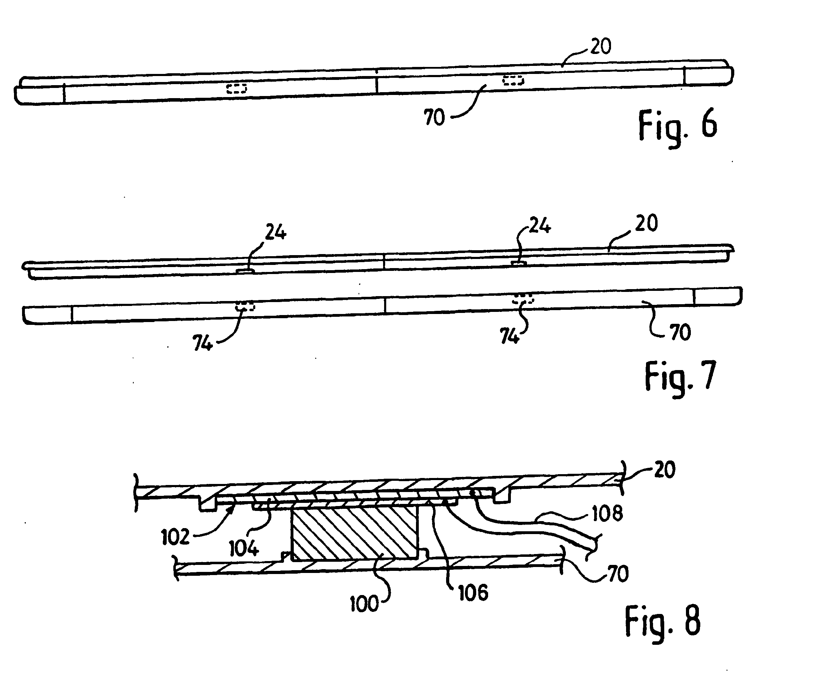

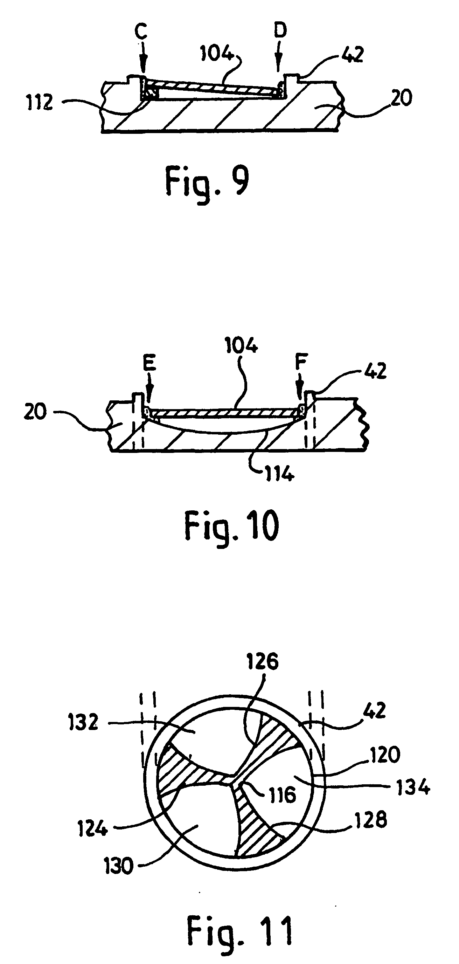

The sensor device 10 will now be described in greater detail with reference to Figures 2 to 11. A plan view of the internal surface of the upper panel 20 is shown in Figure 2, with a plan view of the lower panel 70 shown in Figure 4. Figures 6 and 7 show the interengagement of the upper and lower panels on assembly of the device 10. Figures 9 to 11 show different ways of mounting individual sensors contained within the sensor device.

The upper panel 20 is substantially rectangular in shape and is injection moulded in plastics material, such as ABS, so as to be substantially rigid, or semi-rigid. The panel 20 is moulded with rounded corners 22 as sharp corners could prove dangerous in use. The external surface of the upper panel 20 is smooth, however as shown in Figure 2 the internal surface is provided with a number of integrally moulded features, such as a peripheral rim 23 with lugs 24, upstanding ribs and circular ribs.

The upstanding ribs, labelled by way of example at 30, 32, 34 and 36, provide an interconnecting series of rectangles which strengthen the upper panel 20 and increase its rigidity. The upstanding ribs also ensure that as the upper panel leaves the moulding machine, it remains substantially flat. Circular ribs 38, 40, and 42 are also moulded into the upper panel. Each circular rib provides a guide for the placing of the individual sensors which comprise the sensing means. Thus in Figure 2, five piezo-electric sensors in the form of flat discs are provided, with one larger sensor of diameter 35mm placed within circular rib 42 and the four remaining edge sensors of diameter 27mm each placed within one of the four circular ribs 38 and 40. The larger central sensor 44 is shown in the sectional view of Figure 3. The provision of sensors at both the centre and the corner positions of the upper panel 20 improves the sensitivity of the device 10, particularly when a child rolls to a part of the mattress not directly over the device, as this extends the effective area of the pad over that achieved when using only one central sensor. The use of smaller sensors as the edge sensors provides a cost saving without unduly compromising performance. In addition the sensors are electrically connected in parallel.

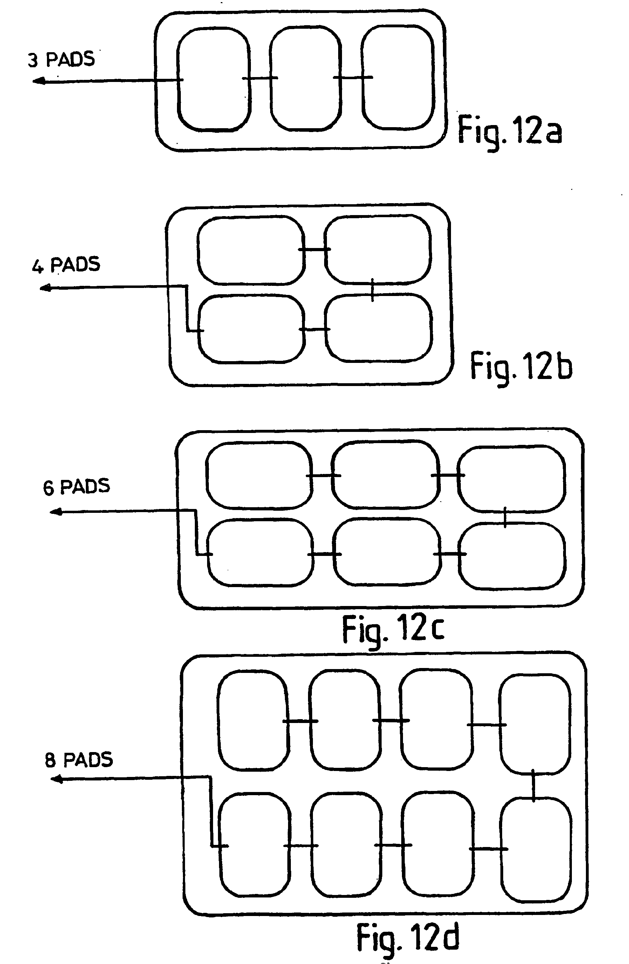

The path of electrical wiring between the sensors is facilitated by the upstanding ribs providing channels 46, 48, 50, 52, and 54 in which the electrical wiring between the individual sensors is guided. Also each circular rib possesses one or more apertures to provide the electrical wires with a connection path to the individual sensors, and the peripheral rim 23 has a cut-out groove 56 through which the electrical wire passes to communicate the combined electrical signal from the sensors to a signal processing unit. A further cut-out groove in the rim 23, typically diametrically opposite groove 56, allows the electrical wire to pass to another sensor device 10 when several sensor devices are connected together in an array as shown in Figure 12.

The upper panel 20 is shown in section in Figure 3, with the internal surface seen in Figure 2 being viewed along the direction of arrow A. The features of the peripheral rim 23, circular rib 42, and piezo-electric sensor 44 are visible.

In Figure 4, the internal surface of the lower panel 70 is shown, with the section along line V-V as seen in Figure 5 showing the internal surface along the direction of arrow B. The lower panel 70 is made from the same moulded plastics material as the upper panel 20 and is thus also substantially rigid, or semi-rigid. As for the upper panel 20, the lower panel 70 is substantially rectangular with the corners of the rectangle being rounded for safety reasons. The panel 70 has a peripheral rim 72 which contains notches 74 and the lip is provided with a cut-out groove 76 which is complementary to groove 56 in the upper panel 20. A further groove, typically diametrically opposite groove 76, is also incorporated and complementary to that described in the upper panel 20.

The lower panel 70 includes a series of integrally moulded protruding areas, as identified by way of example at 78, 80 and 82. As seen in Figure 5, from the outer surface of the lower panel these protruding areas 78, 80 and 82 are viewed as substantially rectangular indentations which act to strengthen the lower panel 70 and improve its rigidity in a similar manner to the upstanding ribs provided in upper panel 20. The positioning of the protruding areas 78, 80 and 82 is such that when the upper panel 20 and the lower panel 70 are brought into interengagement, the protruding areas 78, 80 and 82 fall within the rectangular areas defined on the upper panel 20 by the upstanding ribs.

A series of circular ribs, shown by way of example at 84, 86, 88, 90, 92 and 94, are included on moulding so as to define areas where resilient pads are positioned. Five resilient pads in the form of cylinders and typically made from foam material, are spaced over the lower panel within circular ribs 84, 86, 90, 92 and 94, each pad being placed in an area defined by one circular rib and an example resilient pad 96 can be seen in Figure 5. Up to eleven resilient pads may be used over the lower panel. Corresponding numerals have been used to label features in Figure 5 which are shown in Figure 4.

In Figure 6, the upper panel 20 is shown mounted on the lower panel 70, this being achieved by interengagement of the lugs 24 into the notches 74 provided in the peripheral rim 72 of the lower panel 70. The lugs 24 and notches 74 interengage with a snap-fit connection, the snap-fit allowing guided movement of the upper panel 20 towards and away from the lower panel 70. The interengaging elements 24, 74 thus guide the upper panel in relation to the lower panel whilst ensuring that the two panels 20, 70 always remain in registration. The exploded view of Figure 7 clearly shows the positioning of the lugs 24 and notches 74.

On construction of the sensor device 10, the five piezo-electric sensors are adhered inside the circular ribs 38, 40 and 42 and wired together by passing electrical wires along the channels 46, 48, 50, 52, and 54. The five resilient pads are then adhered in the areas defined on the lower panel 70 by the circular ribs 84, 86, 90, 92 and 94 to provide a plurality of spaced apart resilient mountings opposing the sensors. The upper and lower panels are then brought into interengagement by snapping the lugs 24 and notches 74 together. The corresponding grooves 56 and 76 ensure that an exit aperture for an electrical wire is provided from the interengaged panels.

When the panels are mounted together as in Figure 6, the cylindrical resilient pads act to space apart the panels 20, 70. The five piezo-electric sensors each engage one resilient pad, with any remaining resilient pads which do not engage a sensor acting solely as spacers between the two panels 20, 70. Where a resilient pad engages with a sensor, the resilient pad only contacts a central portion of the area covered by the sensor and does not overlie all the sensor area, as shown in Figure 8 where a resilient pad 100 and piezo-electric sensor 104 are located between upper and lower panels 20, 70, with electrical wires 108 leading from the sensor 104. In normal use, the stiffness of the panels in relation to the resilience of the pads is such that the panels do not contact each other, except where the lugs and notches interengage, and thus the upper panel 20 floats on the lower panel 70. The rigidity of the panels is such that at no time can the upper panel bottom out on the lower panel.

The upper panel 20 and the lower panel 70 are fairly rigid but when a varying force is applied to the upper panel 20, the upper panel 20 flexes downwardly slightly more over its unsupported areas than where it is resiliently supported on the resilient pads 100. Thus as a child moves during breathing and varies its weight distribution over the upper panel, the upper panel flexes slightly and because of the resilient mounting moves towards and away from the lower panel. This slight flexing of the upper panel 20 causes the piezo-electric sensor 104 to be bent slightly, convex upwardly and concave downwardly. At the same time the sensor is compressed due to weight on the upper panel compressing the resilient pads 100, and both bending and compression of the sensor causes the generation of an electric signal between the metal base plate 102, and the active layer 106 of the piezo-electric sensor 104. Thus the varying weight distribution over the upper panel 20 as the child moves is transmitted via the changing bending and compression of the resilient pads 100 to the piezo-electric sensors 104 which generate electrical signals conducted along wires 108. The wires 108 interconnect the piezo-electric sensors in parallel to increase sensitivity through coverage of a greater area. The electrical signals are then analysed to produce information on respiratory movement, movement patterns or heartbeat.

Adhering the piezo-electric sensors directly onto the flat surface of the upper panel 20 within the circular ribs 38, 40 and 42 can cause difficulty with correct positioning of the sensors as uneven distribution of adhesive can disturb flat mounting of the sensors within the circular ribs. Other alternative mounting arrangements for the sensors are possible. For example as shown in Figure 9, a small spacer segment 112 of approximately 0.5mm thickness and 3mm length is provided under one edge of each piezo-electric sensor 104. Adhesive is used to secure the spacer 112 and one edge of the sensor 104 within the circular rib 42 at position C, with a small drop of adhesive placed at position D, within the rib and diametrically opposed to the spacer, to hold the sensor at a second position. The sensor is thus tilted slightly upwards within the rib and consistent mounting of each sensor can be achieved. The spacer can alternatively be provided by a single protruding mound, or pip, of approximately 0.5mm height and integrally moulded at one edge of the area formed by the circular rib. In this arrangement one edge of the sensor rests on the pip with adhesive placed at the pip site and also diametrically opposite it.

A further method of mounting sensors is shown in side view in Figure 10 where a dished recess 114 is provided within the circular rib 42 with the sensor 104 sitting squarely on the outer circumference of the dished recess and adhered in position by a thin layer of adhesive at diametrically opposed positions E and F. This configuration only supports the sensor around its outer annulus and avoids placing any adhesive directly under the centre of the sensor.

As a yet further alternative of mounting the sensor, the flat circular area encompassed by each circular rib can be provided with three protruding mounds, or pips, equally spaced about the circumference of the circular area, so that the sensor sits on the protrusions and is raised by a small distance, for example 0.5mm, off the flat base of the lower panel encompassed by the circular rib. Adhesive is applied to each pip so that the sensor is adhered at three separate circumferentially spaced apart locations.

A similar effect can be produced as shown in Figure 11 by use of a central depression 116 within the circular area 120 provided by the circular rib 42, with three radial arms 124, 126, 128 extending upwardly from the depression to the outer edges of the area 120 so that the sensor sits on three raised areas 130, 132, 134.

Whilst the sensor device described is of particular use in detecting the respiratory movement of a child, it can also be used to detect general movement or heartbeat depending on the sensitivity of the sensors. The sensor device is also suitable for use with larger children or adults when suitably dimensioned, or alternatively several sensor devices may be connected together to provide an enlarged sensing area suitable for detecting adult movement as shown in Figures 12(a), 12(b), 12(c) and 12(d) with arrangements of three, four, six and eight sensor devices connected electrically in parallel. It is envisaged that a plastics sleeve with pockets could be used to hold a plurality of interconnected sensor devices, and thus provide the enlarged sensing area.

## Claims

1. A sensor device (10) for detecting respiratory movement in a person, the device comprising a lower panel (70), an upper panel (20) resiliently mounted on the lower panel (70) and guided for limited relative movement in a direction towards or away from the lower panel, and sensing means (104) positioned between the panels and operative to produce an electrical signal representative of movement of the upper panel (20) with respect to the lower panel (70), **characterised in that** ribs (30, 32, 34, 36) are provided to strengthen the upper panel and improve rigidity.

2. A sensor device (10) for detecting respiratory movement in a person, the device consisting of a lower panel (70), an upper panel (20) resiliently mounted on the lower panel (70) and guided for limited relative movement in a direction towards or away from the lower panel, and sensing means (104) positioned between the panels and operative to produce an electrical signal representative of movement of the upper panel (20) with respect to the lower panel (70), **characterised in that** one of the panels has a projected peripheral lip (72) which locates within a peripheral rim (23) on the other panel to provide the guided relative movement.

3. A sensor device (10) according to claim 1, wherein one of the panels has a projecting peripheral lip (72) which is located within a peripheral rim (23) on the other panel to provide the guided relative movement.

4. A sensor device according to claim 2 or claim 3, wherein the peripheral lip (72) and the peripheral rim (23) include corresponding portions (24, 74) which interengage to provide a snap-fit connection between the upper (20) and lower (70) panel.

5. A sensor device according to claim 2 or claim 4 when dependent on claim 2, wherein ribs (30, 32, 34, 36) are provided to strengthen the upper panel (20) and improve rigidity.

6. A sensor device according to any preceding claim, wherein the device (10) is encased in plastics material which provides substantially waterproof seal about the device so as to prevent ingress of water and urine.

7. A sensor device according to any preceding claim, wherein each of the lower panel (70) and upper panel (20) is moulded from a rigid or semi-rigid plastic material.

8. A sensor device according to claim 7, wherein moulding is performed by injection moulding.

9. A sensor device according to claim 7 or claim 8 when dependent on any one of claims 1 or 5, wherein the ribs (30, 32, 34, 36) are integrally moulded into the upper panel.

10. A sensor device according to any preceding claim, wherein a plurality of resilient pads (100), disposed between the panels and spaced over the overlapping area of the panels, provides the resilient mounting of the upper panel (20) on the lower panel (70), and the sensing means (104) is a plurality of piezo-electric sensors engaged by the pads.

11. A sensor device according to any preceding claim, wherein five sensors are provided, wherein one sensor (44) is attached proximal to the centre of the inner surface of the upper panel (20), and four sensors (104) are each positioned at spaced apart locations near the edge of the inner surface of the upper panel (20).

12. A sensor device according to claim 10 or 11, wherein the sensors (104) are electrically connected in parallel.

13. A sensor device according to any of claims 10 to 12, wherein a spacer (112) is positioned under one edge of each sensor (104) so as to tilt each sensor.

14. A sensor device according to any of claims 10 to 12, wherein a recessed area is provided underneath each sensor (104).

15. A plurality of sensing devices (10) in accordance with any of the preceding claims and electrically connected in parallel to increase sensing area.

16. A system for translating the movement of a person into a signal for monitoring the respiratory movement, comprising a sensor device (10) according to any of the preceding claims, a transmitter (16), and a receiver (18), wherein the electrical signals produced from the sensor device (10) are communicated to the transmitter (16), the transmitter (16) then communicating these signals to the receiver (18).

17. A system according to claim 16, wherein the receiver (18) is remote from the sensor device (10) and transmitter (18).

18. A system according to claim 16 or claim 17, wherein the transmitter (16) is remote from the sensor device (10).

19. A system according to any of claims 17 to 18, wherein the receiver (18) is provided with an LCD display for visual display of information derived from the electrical signals resulting from the sensor device (10) and from other sources.

## Patentansprüche

1. Ein Sensorgerät (10) zum Detektieren einer Atembewegung in einer Person, wobei das Gerät eine untere Platte (70), eine obere Platte (20), die federnd auf der unteren Platte (70) montiert ist und für eine begrenzte Relativbewegung in eine Richtung zu oder weg von der unteren Platte geführt wird, und Messmittel (104) umfasst, die zwischen den Platten positioniert sind und betriebsbereit sind, um ein elektrisches Signal zu erzeugen, das für eine Bewegung der oberen Platte (20) in Bezug auf die untere Platte (70) repräsentativ ist, **dadurch gekennzeichnet, dass** Rippen (30, 32, 34, 36) vorgesehen sind, um die obere Platte zu verstärken und eine Steifigkeit zu verbessern.

2. Ein Sensorgerät (10) zum Detektieren einer Atembewegung in einer Person, wobei das Gerät aus einer unteren Platte (70), einer oberen Platte (20), die federnd auf der unteren Platte (70) montiert ist und für eine begrenzte Relativbewegung in eine Richtung zu oder weg von der unteren Platte geführt wird, und Messmitteln (104) besteht, die zwischen den Platten positioniert sind und betriebsbereit sind, um ein elektrisches Signal zu erzeugen, das für eine Bewegung der oberen Platte (20) in Bezug auf die untere Platte (70) repräsentativ ist, **dadurch gekennzeichnet, dass** eine der Platten eine hervorstehende Umfangslippe (72) besitzt, welche innerhalb eines Umfangsrands (23) an der anderen Platte angeordnet ist, um die geführte Relativbewegung bereitzustellen.

3. Ein Sensorgerät (10) nach Anspruch 1, wobei eine der Platten eine hervorstehende Umfangslippe (72) besitzt, welche innerhalb eines Umfangsrands (23) an der anderen Platte angeordnet ist, um die geführte Relativbewegung bereitzustellen.

4. Ein Sensorgerät nach Anspruch 2 oder Anspruch 3, wobei die Umfangslippe (72) und der Umfangsrand (23) entsprechende Abschnitte (24, 74) umfassen, welche ineinander eingreifen, um eine Schnappverschlussverbindung zwischen der oberen (20) und der unteren (70) Platte bereitzustellen.

5. Ein Sensorgerät nach Anspruch 2 oder Anspruch 4, wenn abhängig von Anspruch 2, wobei Rippen (30, 32, 34, 36) vorgesehen sind, um die obere Platte (20) zu verstärken und eine Steifigkeit zu verbessern.

6. Ein Sensorgerät nach einem der vorhergehenden Ansprüche, wobei das Gerät (10) in Kunststoffmaterial eingeschlossen ist, das eine im Wesentlichen wasserdichte Abdichtung um das Gerät herum bereitstellt, um einen Eintritt von Wasser und Urin zu verhindern.

7. Ein Sensorgerät nach einem der vorhergehenden Ansprüche, wobei sowohl die untere Platte (70) als auch die obere Platte (20) aus einem steifen oder halbsteifen Kunststoff geformt sind.

8. Ein Sensorgerät nach Anspruch 7, wobei das Formen durch Spritzgießen durchgeführt wird.

9. Ein Sensorgerät nach Anspruch 7 oder Anspruch 8, wenn abhängig von einem der Ansprüche 1 oder 5, wobei die Rippen (30, 32, 34, 36) integral in der oberen Platte geformt sind.

10. Ein Sensorgerät nach einem der vorhergehenden Ansprüche, wobei eine Vielzahl von federnden Belägen (100), die zwischen den Platten angeordnet und über den überlappenden Bereich der Platten verteilt sind, die federnde Montage der oberen Platte (20) auf der unteren Platte (70) bereitstellt, und die Messmittel (104) eine Vielzahl von piezoelektrischen Sensoren ist, die mit den Belägen wechselwirken.

11. Ein Sensorgerät nach einem der vorhergehenden Ansprüche, wobei fünf Sensoren vorgesehen sind, wobei ein Sensor (44) nahe an der Mitte der inneren Oberfläche der oberen Platte (20) befestigt ist und vier Sensoren (104) jeweils an voneinander beabstandeten Stellen nahe der Kante der inneren Oberfläche der oberen Platte (20) positioniert sind.

12. Ein Sensorgerät nach Anspruch 10 oder 11, wobei die Sensoren (104) elektrisch parallel geschaltet sind.

13. Ein Sensorgerät nach einem der Ansprüche 10 bis 12, wobei ein Abstandshalter (112) unter einer Kante jedes Sensors (104) positioniert ist, um jeden Sensor schräg zu stellen.

14. Ein Sensorgerät nach einem der Ansprüche 10 bis 12, wobei unter jedem Sensor (104) ein ausgesparter Bereich vorgesehen ist.

15. Eine Vielzahl von Messgeräten (10) gemäß einem der vorhergehenden Ansprüche, die elektrisch parallel geschaltet sind, um den Messbereich zu vergrößern.

16. Ein System zum Umwandeln der Bewegung einer Person in ein Signal zum Überwachen der Atembewegung, umfassend ein Sensorgerät (10) gemäß einem der vorhergehenden Ansprüche, einen Sender (16) und einen Empfänger (18), wobei die elektrischen Signale, welche von dem Sensorgerät (10) erzeugt werden, an den Sender (16) übermittelt werden, wobei der Sender (16) diese Signale dann an den Empfänger (18) übermittelt.

17. Ein System nach Anspruch 16, wobei der Empfänger (18) entfernt von dem Sensorgerät (10) und dem Sender (16) angeordnet ist.

18. Ein System nach Anspruch 16 oder Anspruch 17, wobei der Sender (16) entfernt von dem Sensorgerät (10) angeordnet ist.

19. Ein System nach einem der Ansprüche 17 bis 18, wobei der Empfänger (18) mit einer LCD Anzeige zum visuellen Anzeigen von Information versehen ist, die von den elektrischen Signalen abgeleitet wird, welche von dem Sensorgerät (10) und von anderen Quellen kommen.

## Revendications

1. Dispositif de détection (10) pour détecter un mouvement respiratoire chez une personne, ce dispositif comprenant un panneau inférieur (70), un panneau supérieur (20), monté de manière élastique sur le panneau inférieur (70) et guidé pour permettre un déplacement relatif limité dans un sens de rapprochement ou d'éloignement du panneau inférieur, et des moyens de détection (104) placés entre les panneaux et servant à produire un signal électrique représentant le mouvement du panneau supérieur (20) par rapport au panneau inférieur (70), **caractérisé en ce que** des nervures (30, 32, 34, 36) sont prévues pour renforcer le panneau supérieur et améliorer sa rigidité.

2. Dispositif de détection (10) pour détecter un mouvement respiratoire chez une personne, ce dispositif comprenant un panneau inférieur (70), un panneau supérieur (20), monté de manière élastique sur le panneau inférieur (70) et guidé pour permettre un déplacement relatif limité dans un sens de rapprochement ou d'éloignement du panneau inférieur, et des moyens de détection (104) placés entre les panneaux et servant à produire un signal électrique représentant le mouvement du panneau supérieur (20) par rapport au panneau inférieur (70), **caractérisé en ce que** l'un des panneaux comporte une lèvre périphérique en saillie (72) qui se place à l'intérieur d'un rebord périphérique (23) de l'autre panneau pour permettre le déplacement relatif guidé.

3. Dispositif de détection (10) selon la revendication 1, dans lequel l'un des panneaux comporte une lèvre périphérique en saillie (72) qui se place à l'intérieur d'un rebord périphérique (23) de l'autre panneau pour permettre le déplacement relatif guidé.

4. Dispositif de détection selon la revendication 2 ou la revendication 3, dans lequel la lèvre périphérique (72) et le rebord périphérique (23) comprennent des parties correspondantes (24, 74) qui s'engrènent l'une dans l'autre pour permettre un raccordement par encliquetage entre les panneaux supérieur (20) et inférieur (70).

5. Dispositif de détection selon la revendication 2 ou la revendication 4 lorsqu'elle est dépendante de la revendication 2, dans lequel des nervures (30, 32, 34, 36) sont prévues pour renforcer le panneau supérieur (20) et améliorer sa rigidité.

6. Dispositif de détection selon l'une quelconque des revendications précédentes, dans lequel le dispositif (10) est logé dans une matière plastique qui fournit un joint essentiellement étanche à l'eau autour du.dispositif, pour empêcher toute pénétration d'eau et d'urine.

7. Dispositif de détection selon l'une quelconque des revendications précédentes, dans lequel chacun des panneaux inférieur (70) et supérieur (20) est moulé à partir d'une matière plastique rigide ou semi-rigide.

8. Dispositif de détection selon la revendication 7, dans lequel le moulage est réalisé par moulage par injection.

9. Dispositif de détection selon la revendication 7 ou la revendication 8 lorsqu'elle est dépendante de l'une quelconque des revendications 1 ou 5, dans lequel les nervures (30, 32, 34, 36) sont moulées d'une seule pièce dans le panneau supérieur.

10. Dispositif de détection selon l'une quelconque des revendications précédentes, dans lequel une pluralité de tampons élastiques (100), placés entre les panneaux et répartis sur toute la zone de chevauchement des panneaux, permet le montage élastique du panneau supérieur (20) sur le panneau inférieur (70), et dans lequel les moyens de détection (104) sont une pluralité de capteurs piézoélectriques maintenus par les tampons.

11. Dispositif de détection selon l'une quelconque des revendications précédentes, dans lequel cinq capteurs sont fournis, parmi lesquels un capteur (44) est fixé de manière proximale par rapport au centre de la surface interne du panneau supérieur (20) et quatre capteurs (104) sont placés chacun au niveau d'un emplacement éloigné proche du bord de la surface interne du panneau supérieur (20).

12. Dispositif de détection selon la revendication 10 ou 11, dans lequel les capteurs (104) sont reliés électriquement en parallèle.

13. Dispositif de détection selon l'une quelconque des revendications 10 à 12, dans lequel une entretoise (112) est placée sous un bord de chaque capteur (104) pour incliner chaque capteur.

14. Dispositif de détection selon l'une quelconque des revendications 10 à 12, dans lequel une zone en creux est prévue sous chaque capteur (104).

15. Pluralité de dispositifs de détection (10) selon l'une quelconque des revendications précédentes et reliés électriquement en parallèle pour augmenter la zone de détection.

16. Système de traduction du mouvement d'une personne en un signal permettant de surveiller le mouvement respiratoire, comprenant un dispositif de détection (10) selon l'une quelconque des revendications précédentes, un émetteur (16) et un récepteur (18), dans lequel les signaux électriques produits par le dispositif de détection (10) sont communiqués à l'émetteur (16), l'émetteur (16) communiquant ensuite ces signaux au récepteur (18).

17. Système selon la revendication 16, dans lequel le récepteur (18) est distant du dispositif de détection (10) et de l'émetteur (18).

18. Système selon la revendication 16 ou la revendication 17, dans lequel l'émetteur (16) est distant du dispositif de détection (10).

19. Système selon l'une quelconque des revendications 17 à 18, dans lequel le récepteur (18) est équipé d'un écran LCD pour l'affichage visuel d'informations dérivées des signaux électriques émis par le dispositif de détection (10) et d'autres sources.
